# EUROPEAN PATENT APPLICATION

(11) **EP 4 640 674 A1**
(43) Date of publication of application: **29.10.2025**
(21) Application number: 23906024.7
(22) Date of filing: 20.12.2023
(51) Int. Cl.: C07D 263/32, A61K 31/421, A61P 17/06

(54) **CRYSTAL FORMS OF ETHOXIMOD, PREPARATION METHOD THEREFOR AND USE THEREOF**

(30) Priority: 22.12.2022 CN 202211659300
(71) Applicant: Jiankuan (Suzhou) Biotechnology Co., Ltd, Suzhou, Jiangsu 215000 (CN); Institute of Materia Medica, Chinese Academy of Medical Sciences, Beijing 100050 (CN)
(72) Inventor: WANG, Xiaojian, Suzhou, Jiangsu 215000 (CN); CHEN, Xiaoguang, Suzhou, Jiangsu 215000 (CN); ZHAO, Yanshi, Suzhou, Jiangsu 215000 (CN); WU, Ping, Suzhou, Jiangsu 215000 (CN); JIN, Jing, Suzhou, Jiangsu 215000 (CN); LI, Yan, Suzhou, Jiangsu 215000 (CN); SHENG, Li, Suzhou, Jiangsu 215000 (CN); CHEN, Xin, Suzhou, Jiangsu 215000 (CN)
(74) Representative: Regimbeau
(86) International application number: PCT/CN2023/140373
(87) International publication number: WO 2024/131863

(57) **Abstract**

Provided in the present application are crystal forms of ethoximod, a preparation method therefor and the use thereof. The crystal forms involve a crystal form A and a crystal form B, and the crystal form A and the crystal form B can stably exist, have low hygroscopicity, high solubility, good mechanical stability, low fluidity, relatively low adhesiveness and good compressibility, and thus have medical industrial prospects.

## Description

### TECHNICAL FIELD

The present application belongs to the technical field of medicines and, in particular, relates to crystal forms of ethoximod, a preparation method therefor and use thereof.

### BACKGROUND

Ethoximod is an immunosuppressive agent for an S1P1 receptor and a new drug for treating psoriasis. Ethoximod has a chemical name of 2-amino-2-{2-(4'-(2-ethyloxazol-4-yl)-[1,1'-biphenyl]-4-yl)ethyl}-1,3-propylene glycol hydrochloride and a structural formula as follows:

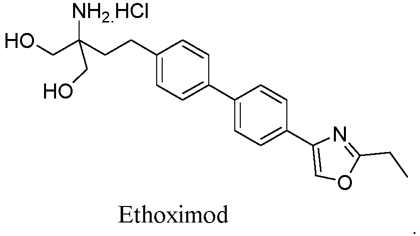

At present, although a preparation method for ethoximod is disclosed in the existing art, no research is performed on crystal forms. If a main drug component used in a preparation process of a drug preparation is a mixed crystal, it may significantly impact the dissolution performance and efficacy of the preparation product. Therefore, the research on the crystal forms of ethoximod is of great significance for the subsequent preparation research and development and the drug treatment effect.

### SUMMARY

The present application provides crystal forms of ethoximod, a preparation method therefor and a use thereof.

In a first aspect, the present application provides a crystal form A of ethoximod. The crystal form A has diffraction peaks with the following 2θ diffraction angles on an X-ray diffraction pattern: 9.295°±0.2°, 14.026°±0.2°, 17.107°±0.2°, 17.774°±0.2°, 21.458°±0.2°, 21.937°±0.2°, 22.396°±0.2°, 26.883°±0.2°, 28.236°±0.2° and 28.534°±0.2°.

In a second aspect, the present application provides a preparation method for the crystal form A of ethoximod described in the first aspect. The preparation method includes the following steps: adding a sample of ethoximod to methanol, heating to dissolve the sample of ethoximod, and evaporating a solvent to obtain the crystal form A.

Preferably, the heating is performed to 50-60 °C, for example, 50 °C, 53 °C, 55 °C, 58 °C or 60 °C.

In a third aspect, the present application provides a crystal form B of ethoximod. The crystal form B has diffraction peaks with the following 2θ diffraction angles on an X-ray diffraction pattern: 9.025°±0.2°, 15.108°±0.2°, 16.213°±0.2°, 17.352°±0.2°, 18.151°±0.2°, 19.143°±0.2°, 21.230°±0.2°, 22.113°±0.2°, 23.537°±0.2°, 24.755°±0.2° and 27.928°±0.2°.

In a fourth aspect, the present application provides a preparation method for the crystal form B of ethoximod described in the third aspect. The preparation method includes the following steps:
adding a crystal form A of ethoximod to a mixed solvent to dissolve the crystal form A of ethoximod until a clear solution is obtained, and spray drying the solution to obtain the crystal form B of ethoximod.

Preferably, the mixed solvent is a mixed solvent of acetone and water or a mixed solvent of ethanol and water.

Preferably, a volume ratio of acetone to water is 1:(0.5-2), for example, 1:0.5, 1:0.6, 1:0.8, 1:1.0, 1:1.3, 1:1.5, 1:1.8 or 1:2.

Preferably, a volume ratio of ethanol to water is 1:(0.1-1), for example, 1:0.1, 1:0.3, 1:0.5, 1:0.8 or 1:1.

In the present application, it is found through research that ethoximod has multiple crystal forms, including but not limited to a crystal form A, a crystal form B, a crystal form C and a crystal form D. The crystal form A and the crystal form B have pharmaceutical industrial application value. Through the comparison in multiple indicators such as stability, solubility, hygroscopicity, morphological researches, powder properties and mechanical properties, it is found that compared with the crystal form B, the crystal form A is simple in preparation, high in efficiency and suitable for industrial production at a low cost with lower hygroscopicity, higher solubility and better physicochemical stability and mechanical stability.

In a fifth aspect, the present application provides a pharmaceutical composition. The pharmaceutical composition includes the crystal form A of ethoximod described in the first aspect and/or the crystal form B of ethoximod described in the third aspect and a pharmaceutically acceptable adjuvant.

In a sixth aspect, the present application provides use of the crystal form A of ethoximod described in the first aspect or the crystal form B of ethoximod described in the third aspect in preparation of an immunosuppressive agent for an S1P1 receptor.

Compared with the existing art, the present application has the beneficial effects below.

The crystal form A and the crystal form B of ethoximod of the present application can exist stably. The crystal form A has lower hygroscopicity, higher solubility, better mechanical stability, relatively low flowability, relatively low adhesion and good compressibility, having a good prospect for pharmaceutical industrialization.

### BRIEF DESCRIPTION OF DRAWINGS

FIG. 1 is an X-ray powder diffraction (XRPD) pattern of a crystal form A of ethoximod.
FIG. 2 is a differential scanning calorimetry (DSC) curve of a crystal form A of ethoximod.
FIG. 3 is a thermogravimetric analysis (TGA) curve of a crystal form A of ethoximod.
FIG. 4 is a Fourier-transform infrared (FT-IR) spectroscopy of a crystal form A of ethoximod.
FIG. 5 is a dynamic vapor sorption (DVS) plot of a crystal form A of ethoximod.
FIG. 6 is an X-ray powder diffraction (XRPD) pattern of a crystal form B of ethoximod.
FIG. 7 is a differential scanning calorimetry (DSC) curve of a crystal form B of ethoximod.
FIG. 8 is a thermogravimetric analysis (TGA) curve of a crystal form B of ethoximod.
FIG. 9 is a Fourier-transform infrared (FT-IR) spectroscopy of a crystal form B of ethoximod.
FIG. 10 is a dynamic vapor sorption (DVS) plot of a crystal form B of ethoximod.
FIG. 11 is an X-ray powder diffraction (XRPD) pattern of a crystal form C of ethoximod.
FIG. 12 illustrates stacked X-ray powder diffraction (XRPD) pattern obtained before and after a crystal form C of ethoximod is dried.
FIG. 13 is a thermogravimetric analysis (TGA) curve of a sample of a crystal form B of ethoximod placed under a condition of 92.5% RH for 30 days.
FIG. 14 is an X-ray powder diffraction (XRPD) pattern of a crystal form D of ethoximod.
FIG. 15 is a differential scanning calorimetry (DSC) curve of a crystal form D of ethoximod.
FIG. 16 is a thermogravimetric analysis (TGA) curve of a crystal form D of ethoximod.
FIG. 17 illustrates stacked X-ray powder diffraction (XRPD) pattern of a crystal form A of ethoximod which is stably placed.
FIG. 18 illustrates stacked X-ray powder diffraction (XRPD) pattern of a crystal form B of ethoximod which is stably placed.
FIG. 19 illustrates stacked X-ray powder diffraction (XRPD) pattern obtained before and after a crystal form A of ethoximod is ground.
FIG. 20 illustrates stacked X-ray powder diffraction (XRPD) pattern obtained before and after a crystal form B of ethoximod is ground.
FIG. 21 illustrates stacked X-ray powder diffraction (XRPD) pattern obtained before and after a crystal form A of ethoximod is compressed.
FIG. 22 illustrates stacked X-ray powder diffraction (XRPD) pattern obtained before and after a crystal form B of ethoximod is compressed.
FIG. 23 illustrates stacked X-ray powder diffraction (XRPD) pattern obtained before and after a crystal form A of ethoximod is subjected to a DVS test.
FIG. 24 illustrates stacked X-ray powder diffraction (XRPD) pattern obtained before and after a crystal form B of ethoximod is subjected to a DVS test.
FIG. 25 illustrates microscopic observation results of a crystal form A of ethoximod with a scale of 20 µm.
FIG. 26 illustrates microscopic observation results of a crystal form B of ethoximod with a scale of 10 µm.

### DETAILED DESCRIPTION

Technical solutions of the present application are further described below through specific examples. Those skilled in the art are to understand that the examples described herein are used for a better understanding of the present application and are not to be construed as specific limitations to the present application.

In the examples of the present application, the instrument used for the X-ray powder diffraction test is the D2 Phaser produced by Bruker. The instrument used for the differential scanning calorimetry test is the differential scanning calorimeter DSC 250 produced by TA Instruments. The instrument used for the thermogravimetric analysis is the thermogravimetric analyzer TGA 55 produced by TA Instruments. The instrument used for the FT-IR test is the Fourier infrared spectrometer ID1-summit produced by Thermo. The instrument used for the dynamic vapor sorption test is the DVS Intrinsic produced by Surface Measurement Systems (SMS). The instrument used for the microscopy is the microscope NJF-120A produced by Suzhou Nanguang Electronic Technology Co., Ltd.

### Example 1 Preparation of a crystal form A of ethoximod

3.5 g sample of ethoximod was weighed and added to a 500 mL glass single-necked flask, and 400 mL methanol was added and heated to 55 °C to dissolve the sample. After the sample was dissolved until a clear solution was obtained, the solution was transferred to a rotary evaporator to evaporate a solvent to obtain 2.9 g solid (with a yield of 82.8%), and subsequently, the obtained solid was subjected to XRPD detection. According to the XRPD detection, the obtained solid was the crystal form A of ethoximod.

Analysis result: HPLC-tested purity: 98.84%.

1H-NMR(400 MHz, DMSO-d6): δ = 8.54 (s, 1H), 7.97 (s, 3H), 7.84 (d, J = 8.3 Hz, 2H), 7.71 (d, J = 8.3 Hz, 2H), 7.65 (d, J = 8.1 Hz, 2H), 7.33 (d, J = 8.0 Hz, 2H), 5.44 (t, J = 5.3 Hz, 2H), 3.57 (d, J = 4.7 Hz, 4H), 2.82 (q, J = 7.6 Hz, 2H), 2.72 - 2.64 (m, 2H), 1.91 - 1.82 (m, 2H), 1.30 (t, J = 7.6 Hz, 3H).

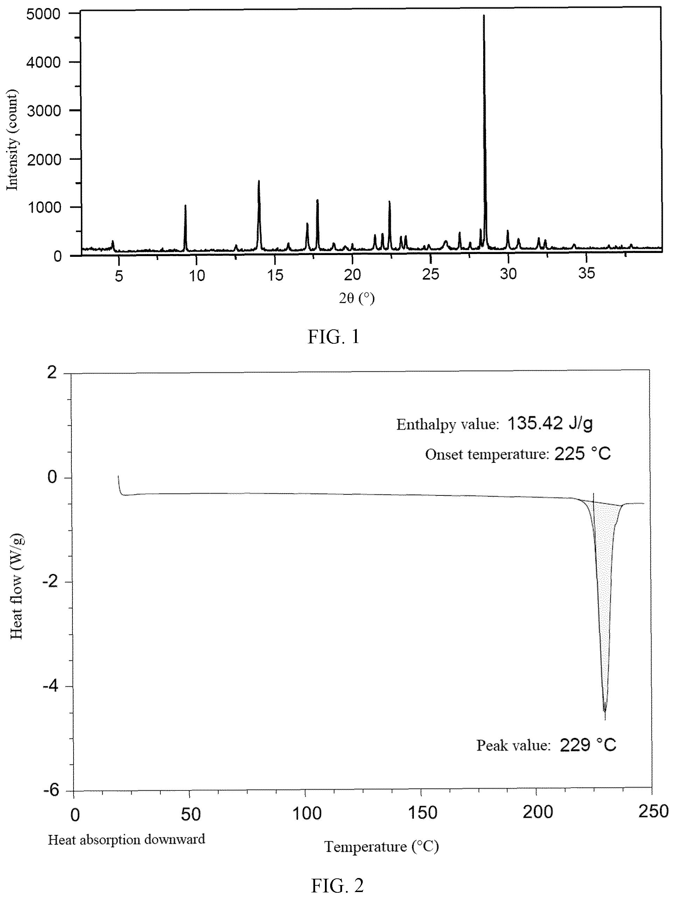

An XRPD pattern of the crystal form A of separated 2-amino-2-{2-(4'-(2-ethyloxazol-4-yl)-[1,1'-biphenyl]-4-yl)ethyl}-1,3-propylene glycol hydrochloride is illustrated in FIG. 1. XRPD parameters in the pattern are summarized in Table 1 below.

**Table 1 XRPD parameters of crystal form A**

| 2θ (°) | D (Å) | Height (counts) | I/I₀ (%) | FWHM (°) |
|---|---|---|---|---|
| 4.307 | 20.4985 | 22 | 0.4 | 0.768 |
| 4.641 | 19.0264 | 219 | 4.2 | 0.096 |
| 7.860 | 11.2395 | 27 | 0.5 | 0.144 |
| 8.587 | 10.2891 | 43 | 0.8 | 0.072 |
| 9.295 | 9.5071 | 974 | 18.7 | 0.072 |
| 11.015 | 8.0262 | 23 | 0.4 | 0.288 |
| 12.557 | 7.0439 | 115 | 2.2 | 0.12 |
| 12.865 | 6.8756 | 26 | 0.5 | 0.144 |
| 14.026 | 6.3091 | 1439 | 27.7 | 0.12 |
| 14.483 | 6.1110 | 9 | 0.2 | 0.096 |
| 15.907 | 5.5668 | 151 | 2.9 | 0.096 |
| 17.107 | 5.1791 | 566 | 10.9 | 0.096 |
| 17.774 | 4.9862 | 1086 | 20.9 | 0.072 |
| 18.816 | 4.7124 | 149 | 2.9 | 0.144 |
| 19.579 | 4.5304 | 73 | 1.4 | 0.24 |
| 20.004 | 4.4351 | 137 | 2.6 | 0.096 |
| 21.458 | 4.1378 | 310 | 6.0 | 0.12 |
| 21.937 | 4.0485 | 340 | 6.5 | 0.12 |
| 22.396 | 3.9665 | 1042 | 20.0 | 0.096 |
| 23.125 | 3.8431 | 280 | 5.4 | 0.12 |
| 23.419 | 3.7955 | 291 | 5.6 | 0.12 |
| 24.609 | 3.6146 | 74 | 1.4 | 0.072 |
| 24.918 | 3.5705 | 83 | 1.6 | 0.144 |
| 26.053 | 3.4174 | 159 | 3.1 | 0.168 |
| 26.883 | 3.3138 | 354 | 6.8 | 0.096 |
| 27.552 | 3.2348 | 142 | 2.7 | 0.144 |
| 28.236 | 3.1580 | 401 | 7.7 | 0.096 |
| 28.534 | 3.1257 | 5203 | 100.0 | 0.072 |
| 29.965 | 2.9796 | 392 | 7.5 | 0.096 |
| 30.651 | 2.9144 | 212 | 4.1 | 0.12 |
| 31.946 | 2.7992 | 229 | 4.4 | 0.096 |
| 32.171 | 2.7802 | 35 | 0.7 | 0.072 |
| 32.376 | 2.7630 | 190 | 3.7 | 0.144 |
| 32.817 | 2.7269 | 51 | 1.0 | 0.096 |
| 33.077 | 2.7060 | 15 | 0.3 | 0.072 |
| 34.284 | 2.6135 | 72 | 1.4 | 0.168 |
| 36.481 | 2.4610 | 45 | 0.9 | 0.096 |
| 36.923 | 2.4325 | 37 | 0.7 | 0.072 |
| 37.274 | 2.4104 | 66 | 1.3 | 0.072 |
| 37.895 | 2.3723 | 80 | 1.5 | 0.12 |

The crystal form A has a differential scanning calorimetry (DSC) curve as shown in FIG. 2. As can be seen from the diagram, when the crystal form A is heated to 225 °C (onset temperature), an endothermic peak appears. The endothermic peak is a melting endothermic peak of the crystal form A.

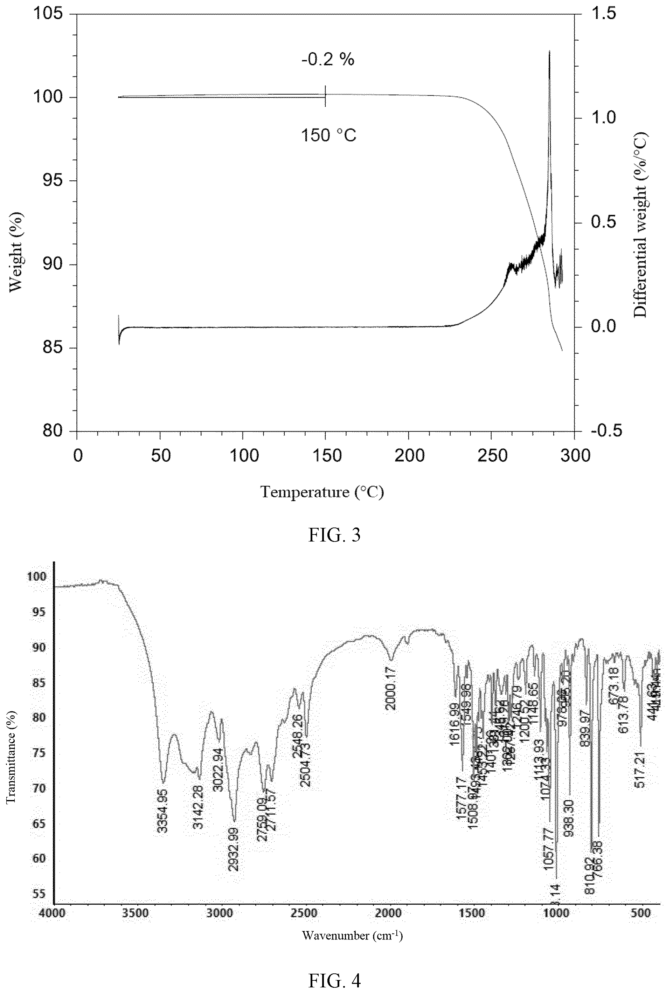

The crystal form A has a thermogravimetric analysis (TGA) curve as shown in FIG. 3. As can be seen from the diagram, when the crystal form A is heated to 150 °C, the crystal form A has a mass loss of about -0.2% (instrument error is ±0.2%).

The crystal form A has a Fourier transform infrared (FTIR) spectrum as shown in FIG. 4. As can be seen from the spectrum, the crystal form A is characterized through attenuated total reflectance-Fourier transform infrared (FTIR) spectroscopy to obtain qualitative data of infrared spectroscopy, proving that the product conforms to a structure of ethoximod.

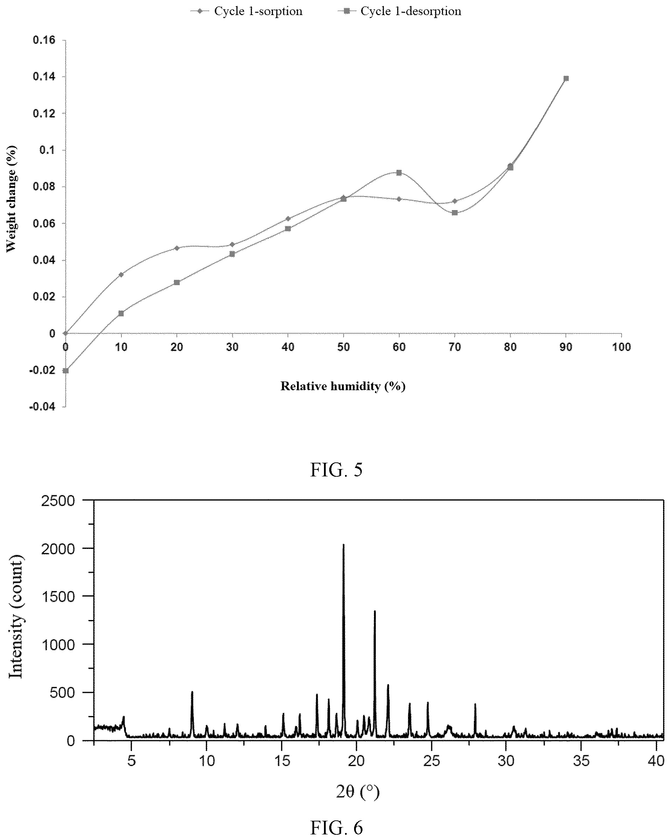

The crystal form A has a dynamic vapor sorption (DVS) plot as shown in FIG. 5. As can be seen from the DVS plot of the crystal form A, the sample has a hygroscopic weight gain of 0.14% with no or almost no hygroscopicity (with vapor sorption at 25 °C/90% RH).

### Example 2 Preparation of a crystal form B of ethoximod

5 g sample of a crystal form A of ethoximod was weighed and added to a 500 mL glass single-necked flask, and 225 mL acetone and 225 mL water were added to dissolve the sample. After the sample was dissolved until a clear solution was obtained, the solution was spray dried to obtain 2.67 g solid (with a yield of 53.4%). According to the XRPD detection, the obtained solid was the crystal form B of ethoximod.

Analysis result: HPLC-tested purity: 98.85%.

¹H-NMR(400 MHz, DMSO-d6): δ = 8.53 (s, 1H), 8.08 - 7.79 (m, 5H), 7.71 (d, J = 8.3 Hz, 2H), 7.65 (d, J = 7.9 Hz, 2H), 7.32 (d, J = 7.9 Hz, 2H), 5.42 (t, J = 5.0 Hz, 2H), 3.56 (d, J = 4.8 Hz, 4H), 2.83 (q, J = 7.6 Hz, 2H), 2.71 - 2.63 (m, 2H), 1.91 - 1.79 (m, 2H), 1.30 (t, J = 7.6 Hz, 3H).

An XRPD pattern of the crystal form B of separated 2-amino-2-{2-(4'-(2-ethyloxazol-4-yl)-[1,1'-biphenyl]-4-yl)ethyl}-1,3-propylene glycol hydrochloride is illustrated in FIG. 6. XRPD parameters are summarized in Table 2.

**Table 2 XRPD parameters of crystal form B**

| 2θ (°) | D (Å) | Height (counts) | I/I₀ (%) | FWHM (°) |
|---|---|---|---|---|
| 4.462 | 19.7897 | 132 | 8.8 | 0.192 |
| 6.416 | 13.7657 | 14 | 1.0 | 0.072 |
| 7.506 | 11.7682 | 73 | 4.8 | 0.072 |
| 9.025 | 9.7902 | 345 | 22.9 | 0.096 |
| 10.032 | 8.8101 | 77 | 5.1 | 0.096 |
| 10.470 | 8.4429 | 49 | 3.2 | 0.072 |
| 11.201 | 7.8932 | 108 | 7.1 | 0.072 |
| 12.088 | 7.3159 | 85 | 5.7 | 0.168 |
| 13.986 | 6.3271 | 16 | 1.0 | 0.288 |
| 15.108 | 5.8597 | 180 | 11.9 | 0.096 |
| 15.954 | 5.5505 | 75 | 5.0 | 0.144 |
| 16.213 | 5.4626 | 173 | 11.5 | 0.096 |
| 16.694 | 5.3063 | 24 | 1.6 | 0.144 |
| 17.352 | 5.1064 | 319 | 21.1 | 0.072 |
| 18.151 | 4.8835 | 289 | 19.2 | 0.096 |
| 18.663 | 4.7506 | 171 | 11.3 | 0.144 |
| 19.143 | 4.6325 | 1510 | 100.0 | 0.072 |
| 19.617 | 4.5217 | 16 | 1.1 | 0.144 |
| 20.082 | 4.4181 | 126 | 8.4 | 0.144 |
| 20.508 | 4.3272 | 152 | 10.1 | 0.12 |
| 20.832 | 4.2606 | 152 | 10.0 | 0.144 |
| 21.230 | 4.1816 | 947 | 62.7 | 0.072 |
| 22.113 | 4.0166 | 391 | 25.9 | 0.12 |
| 23.537 | 3.7767 | 254 | 16.8 | 0.12 |
| 24.755 | 3.5936 | 262 | 17.4 | 0.096 |
| 26.177 | 3.4015 | 75 | 5.0 | 0.48 |
| 27.928 | 3.1922 | 254 | 16.9 | 0.072 |
| 30.557 | 2.9232 | 48 | 3.2 | 0.24 |
| 31.048 | 2.8781 | 12 | 0.8 | 0.096 |
| 31.327 | 2.8531 | 46 | 3.1 | 0.096 |
| 32.495 | 2.7532 | 22 | 1.5 | 0.072 |
| 32.879 | 2.7218 | 55 | 3.6 | 0.096 |
| 34.176 | 2.6215 | 10 | 0.7 | 0.576 |
| 36.023 | 2.4912 | 32 | 2.2 | 0.144 |
| 37.058 | 2.4240 | 54 | 3.6 | 0.096 |
| 37.370 | 2.4044 | 71 | 4.7 | 0.072 |
| 38.571 | 2.3323 | 4 | 0.3 | 0.096 |

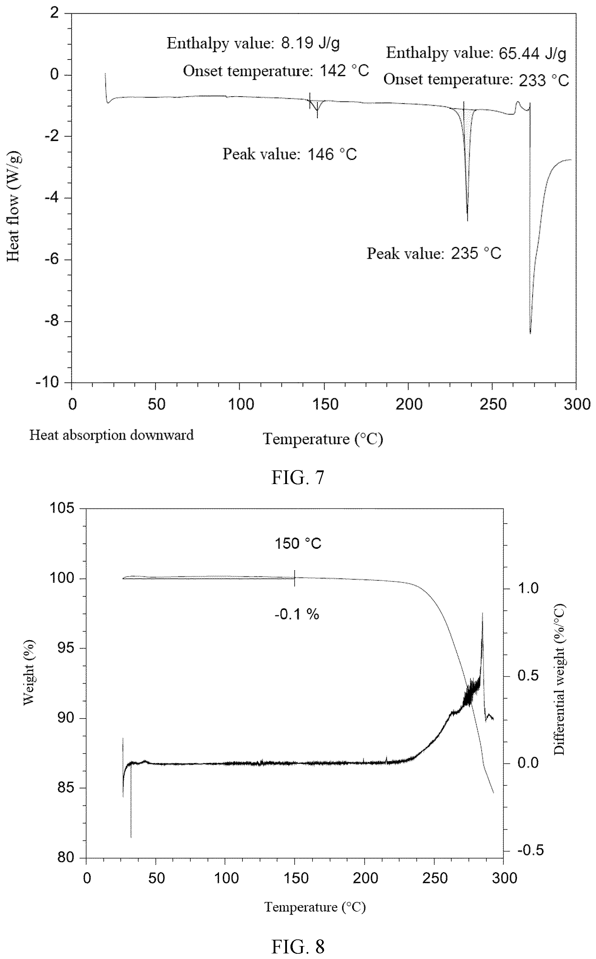

The crystal form B has a differential scanning calorimetry (DSC) curve as shown in FIG. 7. As can be seen from the diagram, when the crystal form B is heated to 142 °C (onset temperature), a first endothermic peak appears; when the crystal form B is heated to 233 °C (onset temperature), a second endothermic peak appears.

The crystal form B has a thermogravimetric analysis (TGA) curve as shown in FIG. 8. When the crystal form B is heated to 150 °C, the crystal form B has a mass loss of about -0.1% (instrument error is ±0.2%). The TGA results indicate that the crystal form B is an anhydrate.

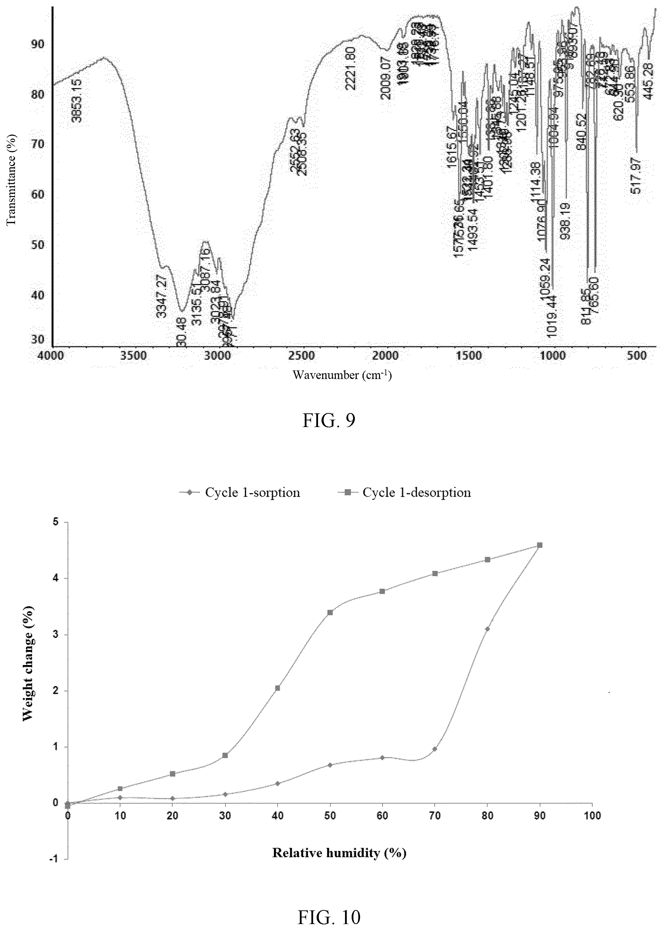

The crystal form B is characterized through attenuated total reflectance-Fourier transform infrared (FTIR) spectroscopy to obtain qualitative data of infrared spectroscopy. A Fourier transform infrared (FTIR) spectrum of the crystal form B is shown in FIG. 9.

The crystal form B has a dynamic vapor sorption (DVS) plot as shown in FIG. 10. As can be seen from the diagram, the sample has a hydroscopic weight gain of 4.6% with hygroscopicity (with vapor sorption at 25 °C/90% RH).

### Example 3 Preparation of a crystal form B of ethoximod

5 g sample of a crystal form A of ethoximod was weighed and added to a 500 mL glass single-necked flask, and 270 mL ethanol and 30 mL water were added to dissolve the sample. After the sample was dissolved until a clear solution was obtained, the solution was cooled to precipitate crystals to obtain 3.1 g solid (with a yield of 62%). According to the XRPD detection, the obtained solid is the crystal form B of ethoximod.

### Example 4 Preparation of a crystal form C of ethoximod

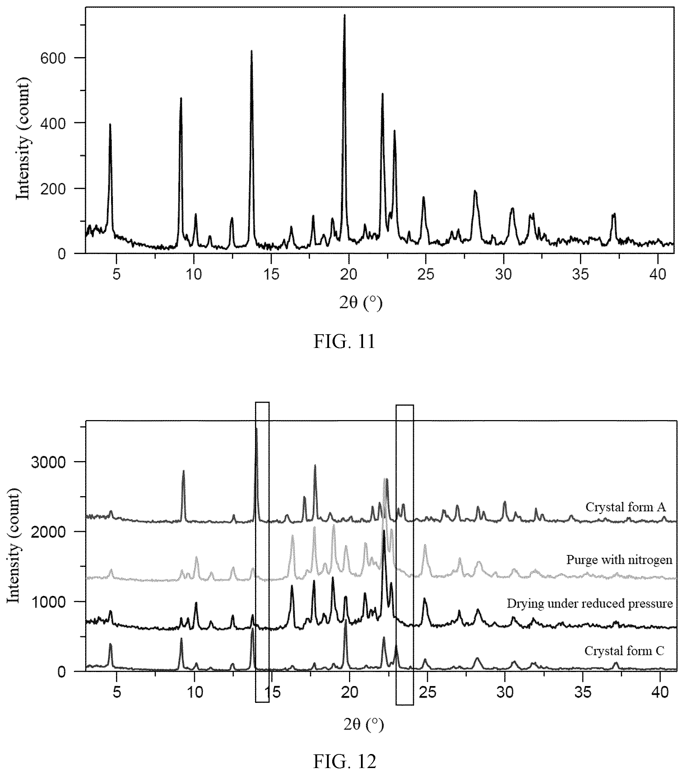

250 mg sample of a crystal form B of ethoximod was weighed and added to a 10 mL glass vial, 5 mL hydrochloric acid aqueous solution (pH = 1.0) was added to form a suspension, and the suspension was placed at room temperature and stirred for 20 h. The XRPD detection results are shown in FIG. 11. The obtained solid is the crystal form C of ethoximod.

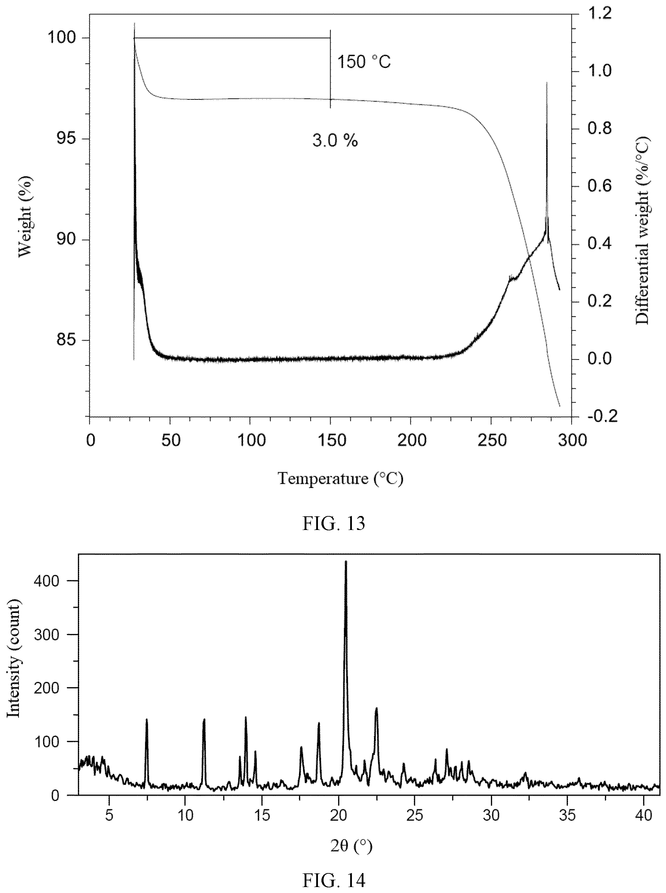

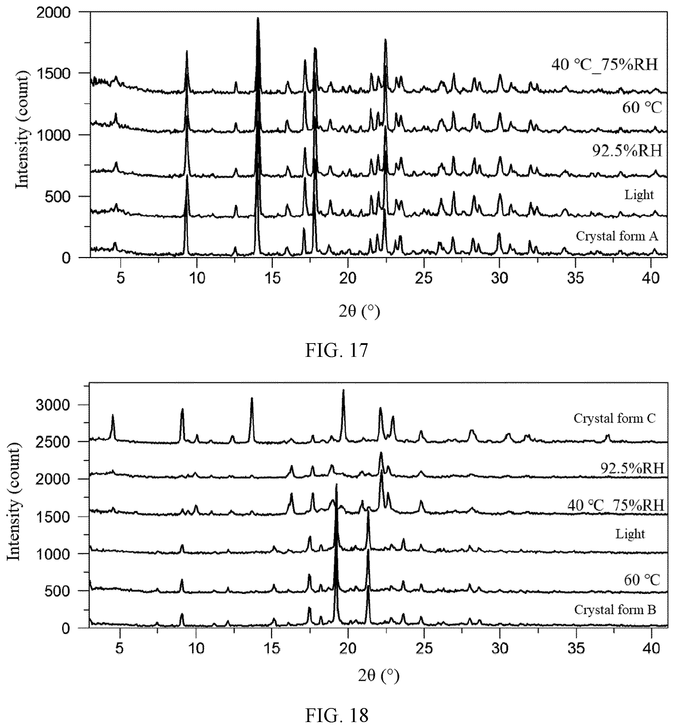

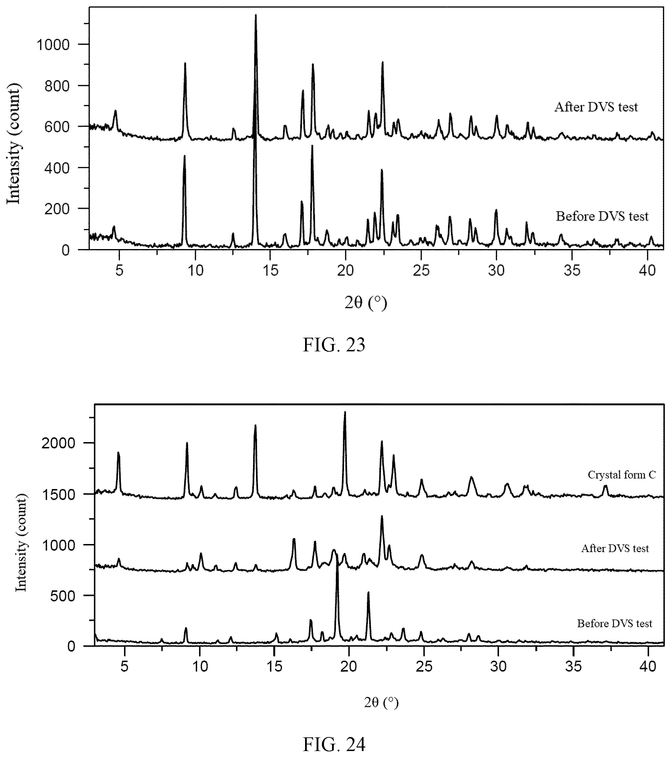

The above sample was placed in vacuum at 50 °C under reduced pressure, dried overnight and subjected to XRPD detection, and as shown in FIG. 12, the solid was partially crystal transformed into a crystal form A of ethoximod. The above sample was purged with nitrogen at room temperature for 3 h and subjected to XRPD detection, and as shown in FIG. 12, the solid was partially crystal transformed into the crystal form A of ethoximod. A sample of the crystal form B of ethoximod after a DVS test was crystal transformed into the crystal form C of ethoximod. An XRPD comparison diagram is shown in FIG. 24. The crystal form B of ethoximod was crystal transformed into the crystal form C of ethoximod after being placed under a condition of 92.5% RH for 30 days. An XRPD comparison diagram is shown in FIG. 18. The sample was subjected to a TGA test. The TGA results are shown in FIG. 13. From the above results, it is speculated that the crystal form C is an unstable channel hydrate that is prone to crystal transformation during drying and placement, making the preparation of a pure phase difficult.

### Analysis results of the dried sample

Purity: 98.81%.
¹H-NMR(400 MHz, DMSO-d6): δ = 8.53 (s, 1H), 7.94 (s, 3H), 7.86-7.82 (m, 2H), 7.74-7.70 (m, 2H), 7.67-7.63 (m, 2H), 7.35-7.31 (m, 2H), 5.43 (t, J = 5.2 Hz, 2H), 3.56 (d, J = 5.1 Hz, 4H), 2.83 (q, J = 7.6 Hz, 2H), 2.71-2.64 (m, 2H), 1.89-1.82 (m, 2H), 1.30 (t, J = 7.6 Hz, 3H).

### Example 5 Preparation of a crystal form D of ethoximod

13 mg sample of a crystal form B of ethoximod was weighed and placed in an aluminum pan. An instrument, TGA 25 of TA Instruments, heated the sample to 180 °C at a temperature ramp rate of 10 °C/min. Under the purge with nitrogen, the sample was at a constant temperature for 40 min and cooled to room temperature to obtain an off-white solid. The XRPD detection results are shown in FIG. 14. The obtained solid is the crystal form D of ethoximod.

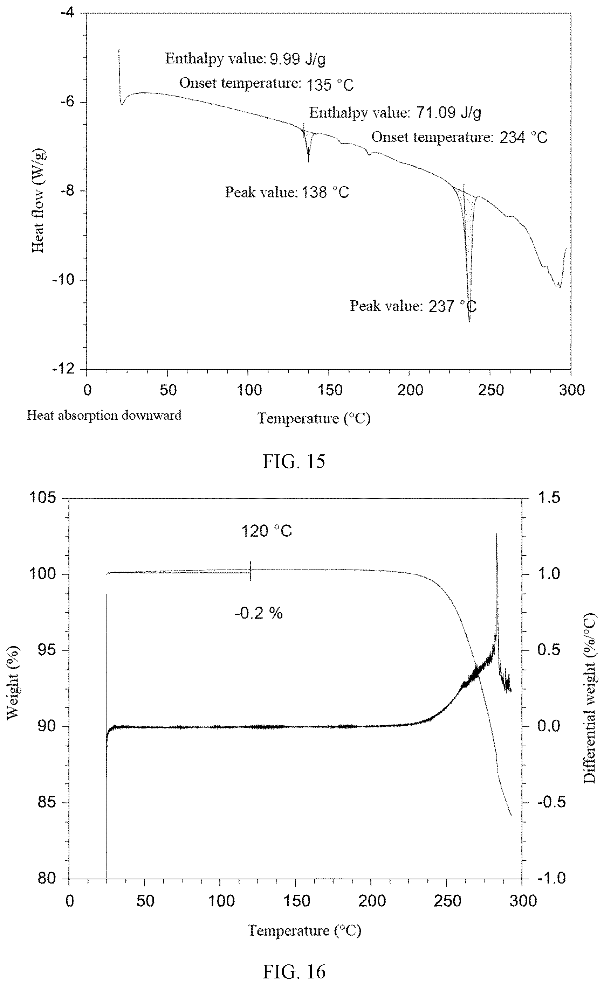

The crystal form D of ethoximod was prepared through the heating experiment and was difficult to magnify. A TGA curve of the crystal form D of ethoximod is shown in FIG. 16. A weight loss of the sample before 150 °C is 1.4%. The heated sample was subjected to ion chromatography detection. A content of chloride ions is 8.32% (a theoretical content of chloride ions is 8.83%). The HPLC purity detection result is 98.70%. The above results indicate that the heating operation does not cause the significant decomposition of ethoximod. In combination with the above, it is speculated that the crystal form D is an anhydrate.

### Analysis results

Purity: 98.70%.
¹H-NMR(400 MHz, DMSO-d6): δ = 8.54 (s, 1H), 8.05-7.80 (m, 5H), 7.75-7.69 (m, 2H), 7.68-7.63 (m, 2H), 7.32 (d, J = 8.2 Hz, 2H), 5.42 (t, J = 5.0 Hz, 2H), 3.56 (d, J = 4.3 Hz, 4H), 2.83 (q, J = 7.6 Hz, 2H), 2.71-2.63 (m, 2H), 1.90-1.80 (m, 2H), 1.30 (t, J = 7.6 Hz, 3H).

### Example 6 Solid-state stability of a crystal form A and a crystal form B of ethoximod

Through the screening of multiple crystal forms of ethoximod, four crystal forms were found in total and named as a crystal form A, a crystal form B, a crystal form C and a crystal form D, respectively. The crystal form C is a hydrate crystal form but is unstable. During drying and placement, the crystal form C is prone to water loss and partially crystal transformed into the crystal form A, making a pure phase difficult to obtain. The crystal form D is an anhydrate crystal form that is obtained after a heating experiment and is difficult to magnify. In conclusion, the crystal form A and the crystal form B of ethoximod were further evaluated.

Samples of the crystal form A and the crystal form B of ethoximod were stored at 60 °C, at 92.5% RH, in light and at 45 °C/75% RH, respectively. Samples were taken before and after storage, and crystal forms were measured through XRPD. The results are shown in Table 3. XRPD comparison spectra of the crystal form A and the crystal form B of ethoximod that are stably placed are shown in FIGS. 17 and 18. The results indicate that the crystal form A can be stable for at least 30 days under the conditions of 60 °C, 92.5% RH, light and 45 °C/75% RH, while the crystal form B can be stable for at least 30 days under the conditions of 60 °C and light but crystal transformed into the crystal form C after being placed for 5 days under the conditions of 92.5% RH and 45 °C/75% RH.

After the crystal form A is stored under the conditions of 40 °C/75% RH, 60 °C and 92.5% RH for 30 days, no significant change occurs in the main purity of the crystal form A, while after the crystal form A is stored under the condition of light for 30 days, the main purity of the crystal form A is slightly decreased. After the crystal form B is stored under the conditions of 40 °C/75% RH, 60 °C and 92.5% RH for 30 days, no significant change occurs in the main purity of the crystal form B, while after the crystal form B is stored under the condition of light for 30 days, the main purity of the crystal form B is decreased to a certain extent.

**Table 3 Comparison of solid-state stability of crystal form A and crystal form B**

| **Storage Condition** | **Crystal Form/Purity (A%)** | |
|---|---|---|
| | **Before Storage** | **After Storage** |
| At 40 °C/75% RH, 30 days exposed | crystal form A main purity: 98.84 maximum single impurity: 0.40 | crystal form A main purity: 98.88 maximum single impurity: 0.35 |
| At 60 °C, 30 days exposed | | crystal form A main purity: 98.82 |
| | | maximum single impurity: 0.35 |
| In light, 30 days exposed | | crystal form A main purity: 97.79 |
| | | maximum single impurity: 0.34 |
| At 92.5% RH, 30 days exposed | | crystal form A main purity: 98.86 |
| | | maximum single impurity: 0.35 |
| At 40 °C/75% RH, 30 days exposed | crystal form B main purity: 98.85 | crystal form C main purity: 98.82 |
| | | maximum single impurity: 0.34 |
| At 60 °C, 30 days exposed | | crystal form B main purity: 98.61 |
| | | maximum single impurity: 0.36 |
| In light, 30 days exposed | maximum single impurity: 0.33 | crystal form B main purity: 91.81 |
| | | maximum single impurity: 1.57 |
| At 92.5% RH, 30 days exposed | | crystal form C main purity: 98.83 |
| | | maximum single impurity: 0.36 |

### Example 7 Investigation of dynamic solubility of a crystal form A and a crystal form B of ethoximod

Samples of the crystal form A and the crystal form B of ethoximod were taken and prepared into saturated solutions at 37 °C in Fasted State Simulated Intestinal Fluid (FaSSIF, pH = 8.0), Fed State Simulated Intestinal Fluid (FeSSIF, pH = 5.0), Simulated Gastric Fluid (SGF, pH = 1.0), a hydrochloric acid aqueous solution (pH = 1.0), an acetic acid-sodium acetate buffer system (pH = 4.0), a phosphate buffer system (pH = 6.8) and water. After 1 h/2 h/4 h/24 h, filtration was performed to obtain solutions, and actual concentrations of the samples in the solutions were measured through high-performance liquid chromatography (HPLC) (diluting the filtrates as needed). Crystal forms of the solids before and after the experiment were characterized through XRPD to check whether phase transition had occurred. The experiment results are shown in Table 4.

**Table 4 Comparison of solubility of crystal form A and crystal form B**

| Medium | Sampling Point | | 1 h | 2 h | 4 h | 24 h |
|---|---|---|---|---|---|---|
| pH = 1.0 | crystal form A | solubility (mg/mL) | 0.28 | 0.28 | 0.29 | 0.38 |
| | | crystal form | A | A | A | A |
| | crystal form B | solubility (mg/mL) | 0.30 | 0.41 | 0.32 | 0.36 |
| | | crystal form | C | C | C | C |
| pH = 4.0 | crystal form A | solubility (mg/mL) | 2.49 | 2.48 | 2.74 | 2.59 |
| | | crystal form | A | A | A | A |
| | crystal form B | solubility (mg/mL) | 1.45 | 1.78 | 1.93 | 2.61 |
| | | crystal form | C | C | C | C |
| pH = 6.8 | crystal form A | solubility (mg/mL) | 0.0002 | 0.00007 | 0.00014 | 0.00076 |
| | | crystal form | free alkali | free alkali | free alkali | free alkali |
| | crystal form B | solubility (mg/mL) | 0.0001 | lower than limit of detection | lower than limit of detection | 0.0019 |
| | | crystal form | free alkali | free alkali | free alkali | free alkali |
| SGF | crystal form A | solubility (mg/mL) | 0.24 | 0.26 | 0.26 | 0.27 |
| | | crystal form | A | A | A | A |
| | crystal form B | solubility (mg/mL) | 0.29 | 0.28 | 0.32 | 0.36 |
| | | crystal form | C | C | C | C |
| FaSSIF | crystal form A | solubility (mg/mL) | 0.01 | 0.009 | 0.011 | 0.026 |
| | | crystal form | A+free alkali | A+free alkali | A+free alkali | A+free alkali |
| | crystal form B | solubility (mg/mL) | 0.015 | 0.01 | 0.008 | 0.021 |
| | | crystal form | free alkali | free alkali | free alkali | free alkali |
| FeSSIF | crystal form A | solubility (mg/mL) | 1.54 | 2.00 | 2.52 | 2.67 |
| | | crystal form | A | A | A | A |
| | crystal form B | solubility (mg/mL) | 1.48 | 1.63 | 1.21 | 1.67 |
| | | crystal form | C | C | C | C |
| water | crystal form A | solubility (mg/mL) | 2.08 | 2.15 | 2.14 | 2.26 |
| | | crystal form | A | A | A | A |
| | crystal form B | solubility (mg/mL) | 1.69 | 1.65 | 2.50 | 2.16 |
| | | crystal form | C | C | C | C |

The results indicate that in the FaSSIF, the FeSSIF, the hydrochloric acid aqueous solution (pH = 1.0), the phosphate buffer system (pH = 6.8) and the water, the solubility of the crystal form A is slightly higher than that of the crystal form B and the crystal form B has already been subjected to crystal transformation in the solvents. This result indicates that the crystal form A is an advantageous crystal form suitable for drug development.

### Example 8 Investigation of equilibrium solubility of a crystal form A and a crystal form B of ethoximod

The crystal form A and the crystal form B of ethoximod were prepared into solutions at room temperature with ethanol, acetone and ethyl acetate. After equilibrium for 24 h, filtration was performed to obtain saturated solutions, and contents of the samples in the saturated solutions were measured through high-performance liquid chromatography (HPLC) (diluting the filtrates as needed). Crystal forms of the solids before and after the experiment were characterized through XRPD to check whether phase transition had occurred. The experiment results are shown in Table 5. The results indicate that in acetone and ethyl acetate at room temperature, the solubility of the crystal form A is slightly higher than that of the crystal form B and the crystal form B has already been subjected to crystal transformation in the media.

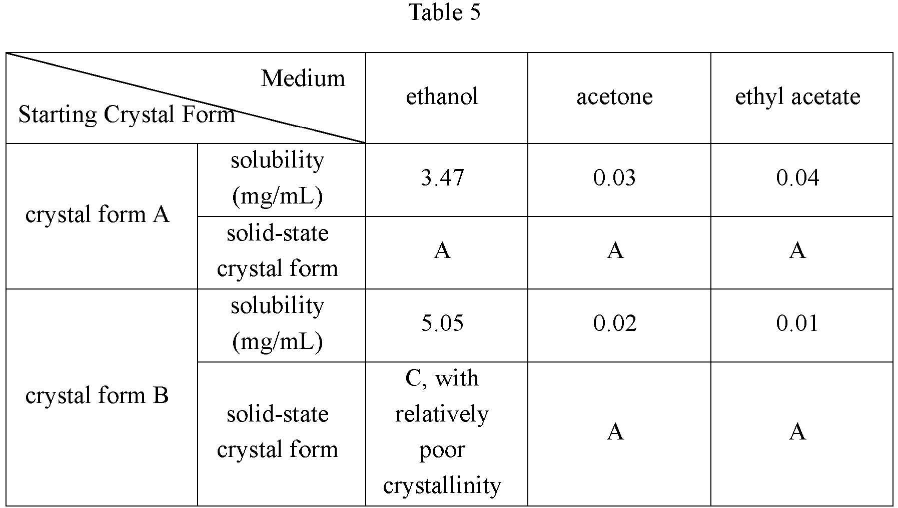

### Example 9 Morphological researches of a crystal form A and a crystal form B of ethoximod

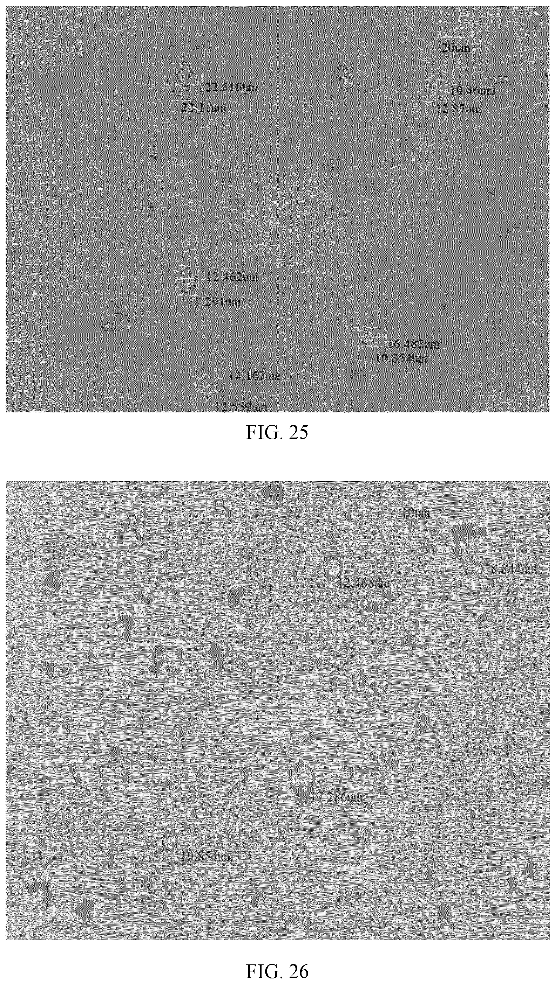

Samples of the crystal form A and the crystal form B of ethoximod were subjected to microscopic morphological researches. The morphology and particle sizes of the crystal form A and the crystal form B were observed under a 40× objective lens of an optical microscope. The microscopic observation results are shown in FIGS. 26 and 27. The crystal form A is irregular and plate-shaped with a raw material particle size of below 35 µm and a long/short diameter ratio of 1 to 2. The crystal form B aggregates and becomes spherical with a raw material particle size of below 20 µm.

### Example 10 Mechanical stability of a crystal form A and a crystal form B of ethoximod

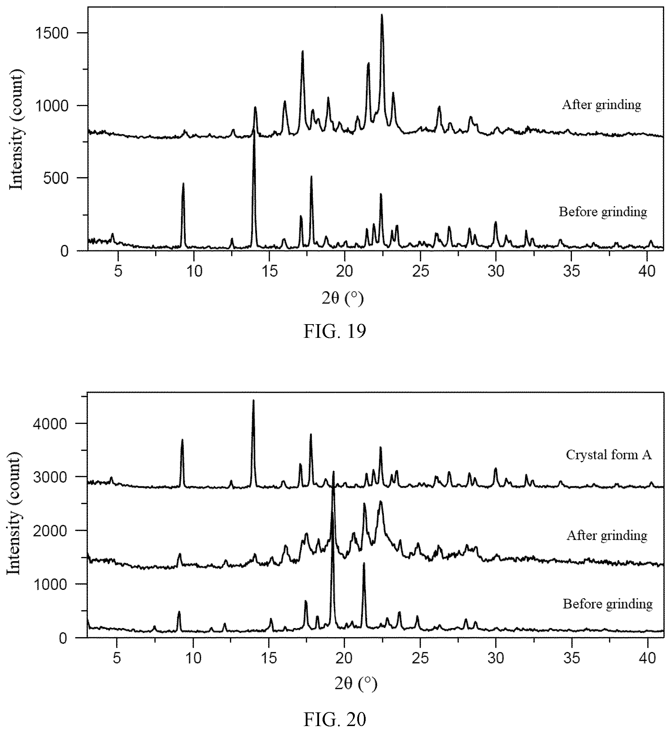

50 mg crystal form A of ethoximod and 50 mg crystal form B of ethoximod were weighed and ground clockwise with an agate mortar for 5 min, and the samples before and after the grinding were subjected to an XRPD test. The results are shown in FIGS. 19 and 20. The XRPD results indicate that the crystal form A remains unchanged after the grinding while the crystal form B is partially crystal transformed into a crystal form C after the grinding. The mechanical stability of the crystal form A is superior to that of the crystal form B.

### Example 11 Hygroscopicity of a crystal form A and a crystal form B of ethoximod

Samples of the crystal form A and the crystal form B of ethoximod were taken for a dynamic vapor sorption test. The results are shown in FIGS. 5 and 10. The test results indicate that the crystal form A has no or almost no hygroscopicity (with a vapor sorption weight gain of 0.14% at 24.3 °C and 90% RH) while the crystal form B has hygroscopicity (with a vapor sorption weight gain of 4.6% at 25.0 °C and 90% RH). After the dynamic vapor sorption test, the crystal form A is not subjected to crystal transformation, while the crystal form B is crystal transformed into a crystal form C. The XRPD comparison results are shown in FIGS. 23 and 24.

### Example 12 Flowability of a crystal form A and a crystal form B of ethoximod

Powder properties of active pharmaceutical ingredients affect the flowability of the active pharmaceutical ingredients. The crystal form A and the crystal form B of ethoximod was subjected to angle of repose and Hausner ratio tests to compare the flowability of the crystal form A and the crystal form B of ethoximod. The flowability evaluation results are shown in Table 7. The crystal form A and the crystal form B are comparable in flowability.

### Methods for the angle of repose test

In this experiment, angles of repose of active pharmaceutical ingredients of the two crystal forms were measured through an injection method in combination with a residual cone method, and the flowability of the active pharmaceutical ingredients of the two crystal forms was analyzed. Specific operations are as follows: powders were injected into a center of a disc with a limited diameter, the injection was stopped until materials on an oblique side of a powder accumulation layer automatically flew out along an edge of the disc, a height (H) of a cone was measured, and the angle of repose was calculated according to the diameter (D = 2r) of the disc with tanQ = H/r. The angle of repose test results are shown in Table 6.

### Method for a bulk density and tap density test

In this experiment, the measurement was performed by using a tap instrument according to a fixed volume method. Specific operations are as follows: the weighed powders were loaded into a 50 mL graduated cylinder, and the graduated cylinder was fixed on a support; the tap instrument was set to vibrate 55±5 times per minute for 6-8 min; a change in a volume of the powders in the measuring cup was measured. A ratio of a mass of the powders to a volume (50 mL) of the materials before the tap is the bulk density of the powders, and a ratio of the mass of the powders to a volume of the materials after the tap is the tap density of the powders.

Methods for converting Hausner ratio and compressibility are as follows, and the description of Hausner ratio and flowability is shown in Table 8:

**Table 6 Physical properties of crystal form A and crystal form B**

| Crystal Form | H (cm) | D (cm) | Angle of Repose (°) | Average of Angles of Repose (°) |
|---|---|---|---|---|
| crystal form A | 1.5 | 2.0 | 56.3 | 57.2 |
| | 1.6 | 2.0 | 58.0 | |
| crystal form B | 1.2 | 2.0 | 50.2 | 52.3 |
| | 1.4 | 2.0 | 54.5 | |

| | | | | |
|---|---|---|---|---|
| Hausner ratio of materials = tap density/bulk density compressibility of materials = (tap density - bulk density)/tap density | | | | |

**Table 7 Flowability of crystal form A and crystal form B**

| Crystal Form | Average of Angles of Repose (°) | Bulk Density (g/cm³) | Tap Density (g/cm³) | Hausner Ratio | Compressibility |
|---|---|---|---|---|---|
| crystal form A | 57.2 | 0.2578 | 0.5604 | 2.2 | 54.0 |
| crystal form B | 52.3 | 0.0742 | 0.1953 | 2.6 | 62.0 |

**Table 8 Relationship between Hausner ratio and flowability**

| Hausner | Description of Flowability |
|---|---|
| 1.00-1.11 | very good flowability |
| 1.12-1.18 | good flowability |
| 1.19-1.25 | fair or moderate flowability |
| 1.26-1.34 | acceptable flowability |
| 1.35-1.45 | poor flowability |
| 1.46-1.59 | very poor flowability |

### Example 13 Adhesion of a crystal form A and a crystal form B of ethoximod

Each of the crystal form A and the crystal form B of ethoximod was taken and put into a ziplock bag with a fixed size. A seal was sealed, and the ziplock bag was flipped at a rotation speed of 5 rpm for 10 min. A direction was changed, and the ziplock bag continued to be flipped at a fixed rotation speed for 10 min. Raw materials were poured out from the bag, a mass of the remaining raw materials was weighed accurately, and an adhesion rate was calculated.

### Method for calculating the adhesion rate

adhesion rate = (mass of initial materials - mass of remaining materials)/contact surface area

The adhesion results of the crystal form A and the crystal form B of ethoximod are shown in Table 9. As can be seen from the results, the crystal form A and the crystal form B are comparable in adhesion rate.

**Table 9**

| Crystal Form | Empty Bag (g) before Test | Bag Weight (g) after Test | Surface Area (m²) of Bag | Weight of API (g) Adsorbed in Bag | Adhesion Amount Per Unit Area (g/m²) |
|---|---|---|---|---|---|
| crystal form A | 0.6878 | 0.7931 | 65 | 0.1053 | 0.002 |
| crystal form B | 0.6858 | 0.7593 | 65 | 0.0735 | 0.001 |

### Example 14 Compressibility of a crystal form A and a crystal form B of ethoximod

The crystal form A and the crystal form B of ethoximod were taken for preparation tableting evaluation. In this experiment, samples were subjected to tableting under the same pressure by using a punch die with a diameter of 6 mm, radial crushing forces F, diameters D and thicknesses L of the tablets were measured after the elastic recovery of the tablets, and the tensile strength of the tablets was calculated according to the following formula: tensile strength Ts = 2F/ΠDL.

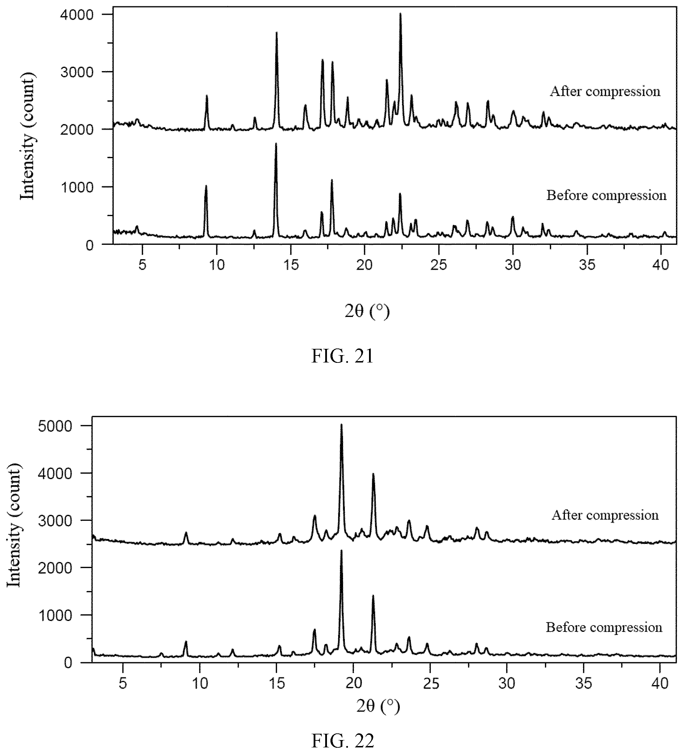

The compressibility evaluation results of the crystal form A and the crystal form B of ethoximod are shown in Table 10, and the XRPD results before and after the compression are shown in FIGS. 21 and 22. As can be seen from the results, a sticking degree of the crystal form A is lower than that of the crystal form B, indicating that the crystal form A is more suitable for preparing a solid preparation.

**Table 10**

| No. | Component | Thickness L (mm) | Diameter D (mm) | Radial Crushing Force F (Hardness/N) | Experimental Phenomenon |
|---|---|---|---|---|---|
| 1 | crystal form A | 3.34 | 6 | 5 | slight sticking |
| | | 3.36 | 6 | 3 | slight sticking |
| 2 | crystal form B | 3.02 | 6 | 11 | relatively severe sticking |
| | | 2.80 | 6 | 4 | relatively severe sticking |

The applicant has stated that although the crystal forms of ethoximod, the preparation method therefor and the use thereof in the present application are described through the above-mentioned examples, the present application is not limited to the above-mentioned processes and steps, which means that the implementation of the present application does not necessarily depend on the above-mentioned processes and steps. It should be apparent to those skilled in the art that any improvements made to the present application, equivalent replacements of raw materials selected in the present application and additions of adjuvant ingredients thereof, and selections of specific methods, etc., all fall within the protection scope and the disclosed scope of the present application.

## Claims

1. A crystal form A of ethoximod having diffraction peaks with the following 2θ diffraction angles on an X-ray diffraction pattern: 9.295°±0.2°, 14.026°±0.2°, 17.107°±0.2°, 17.774°±0.2°, 21.458°±0.2°, 21.937°±0.2°, 22.396°±0.2°, 26.883°±0.2°, 28.236°±0.2° and 28.534°±0.2°.

2. A preparation method for the crystal form A of ethoximod according to claim 1, comprising the following steps: adding a sample of ethoximod to methanol, heating to dissolve the sample of ethoximod, and evaporating a solvent to obtain the crystal form A.

3. The preparation method according to claim 2, wherein the heating is performed to 50-60 °C.

4. A crystal form B of ethoximod having diffraction peaks with the following 2θ diffraction angles on an X-ray diffraction pattern: 9.025°±0.2°, 15.108°±0.2°, 16.213°±0.2°, 17.352°±0.2°, 18.151°±0.2°, 19.143°±0.2°, 21.230°±0.2°, 22.113°±0.2°, 23.537°±0.2°, 24.755°±0.2° and 27.928°±0.2°.

5. A preparation method for the crystal form B of ethoximod according to claim 4, comprising the following steps:
adding a crystal form A of ethoximod to a mixed solvent to dissolve the crystal form A of ethoximod to a clear solution, and spray drying the solution to obtain the crystal form B of ethoximod.

6. The preparation method according to claim 5, wherein the mixed solvent is a mixed solvent of acetone and water or a mixed solvent of ethanol and water.

7. The preparation method according to claim 6, wherein a volume ratio of acetone to water is 1:(0.5-2).

8. The preparation method according to claim 6, wherein a volume ratio of ethanol to water is 1:(0.1-1).

9. A pharmaceutical composition comprising the crystal form A of ethoximod according to claim 1 and/or the crystal form B of ethoximod according to claim 4 and a pharmaceutically acceptable adjuvant.

10. Use of the crystal form A of ethoximod according to claim 1 or the crystal form B of ethoximod according to claim 4 in preparation of an immunosuppressive agent for an S1P1 receptor.
